# EUROPEAN PATENT APPLICATION

(11) **EP 1 413 885 A2**
(43) Date of publication of application: **28.04.2004**
(21) Application number: 04002138.8
(22) Date of filing: 01.04.1998
(51) Int. Cl.: G01N 33/50

(54) **Use and screening method for an aberrant gene product-operating substance**

(30) Priority: 02.04.1997 JP 8375897
(62) Divisional of application: 98911161.2
(71) Applicant: Takeda Chemical Industries, Ltd., Osaka-shi, Osaka 541-0045 (JP)
(72) Inventor: Fujino, Masahiko, Takarazuka-shi, Hyogo 665-0805 (JP)
(74) Representative: Helbing, Jörg, Dr.

(57) **Abstract**

Provided are uses for a substance capable of operating an aberrant gene product, which is prepared by a new drug creation technology that focuses on the cause of disease using information obtained from analysis of structural genes associated with diseases. Thus, the present invention includes (1) a pharmaceutical composition containing a substance capable of operating an aberrant gene product, (2) a method of using said substance for treatment of a disease caused by said product, (3) a screening method for said substance, and (4) a method of preparing a drug for treatment of a disease caused by said product.

## Description

### Technical Field

The present invention relates to a screening method for a substance capable of operating an aberrant gene product and use thereof. More specifically, the present invention relates to a method of creating drugs based on the relationship of a gene and the cause of disease," using information obtained from analysis of structural genes associated with human diseases, e.g., information that serves to elucidate the causal relationship between a gene polymorphism and a disease, and to a use of a substance capable of operating an aberrant gene product prepared on the basis of said new method of pharmaceutical creation.

### Background Art

Conventional drug screening technology is based mainly on research into the pathologic mechanism of disease in an attempt to explore the feasibility of new drug development from the viewpoint of action mechanisms. This pathologic mechanism represents the outcome, rather than the cause, of pathogenesis. For this reason, even a lead compound obtained from a screening system has often been withdrawn from development due to a failure to obtain the desired clinical effect, clinical manifestation of unexpected toxicity, or another such obstacle. Genome research has drawn attention as a new technology since 1990. The vast genetic information obtained by analytical research into genetic information widely involved in biology and medicine is expected to provide clues to the elucidation of the causal relationship between gene polymorphism and diseases, and to enable the identification and creation of drug based on the cause of pathogenesis.

The process for development of drugs, derived from this genetic information, that act on the cause of pathogenesis, is set forth in the step shown bhelow. Specifically, this drug development strategy comprises:
- determining of the sequence and function of a disease-associated gene,
- determining a gene correlated with the cause of pathogenesis and performing functional analysis of genetic information, and
- selecting candidate drugs on the basis of information on the gene that is the cause of pathogenesis.

Presently, the drug creation approach based on genetic information associated with the genome project is in the initial stage of determining the sequence and function of a disease-associated gene, with efforts being undertaken all over the world to elucidate the relationship between diseases and genetic abnormalities.

Although some disease-associated genes have been reported in Alzheimer's disease and hypertension it will take, considerable time will be taken to completely elucidate the pathology of these diseases because various risk factors remain to be clarified. In obesity, the cause of pathogenesis may soon be elucidated; an anti-obesity drug may soon be developed in the near future. In non-insulin-dependent diabetes mellitus, pathogenetic factors for diabetes may soon be demonstrated at the gene level. In heart diseases, a gene associated with myocardial infarction has already been discovered. These and other achievements suggest it very likely that a drug with a new mechanism of action will be found in near future. In cancer research, efforts to discover genes for etiologic or risk factors have steadily yielded good results. It should be noted, however, that different types of cancer involve high diversity such that more findings must be obtained before the cause of pathogenesis of each type is completely clarified.

Most patients with Alzheimer's disease (AD) are solitary cases with unknown hereditary traits. However, there are a small number of cases of familial Alzheimer's disease (FAD) showing autosomal dominant inheritance. The demonstration of the etiologic gene for such FAD would lead to the elucidation of the pathology of solitary AD and subsequent development of a therapeutic drug. Studies regarding the FAD gene have recently been conducted extensively. As a result, it has been shown to date that there is close linkage of the 14th chromosome in many families with early onset FAD, and that there is an abnormality in the β amyloid precursor protein gene, located in the 21st chromosome, in a few families.

In contrast, for solitary Alzheimer's disease, which accounts for the majority of cases of Alzheimer's disease, or familial tardive Alzheimer's disease, the molecular genetic etiology remained unknown until recent years. In recent years, however, studies have been performed based on the hypothesis that these diseases may be multiple-factor hereditary diseases involved by a number of genetic risk factors. In 1993, Corder et al. reported on APO E4, a polymorphism of the apolipoprotein E gene, as a genetic risk factor for Alzheimer's disease (Science, 261, p. 921, 1993). They analyzed the APO Es in a large number of Alzheimer's disease patients and reported out that APO E4 among those genetic polymorphism for protein is a genetic risk factor. According to their report, the prevalence of APO E4 is significantly higher in the familial delayed Alzheimer's disease patient group than in the normal healthy subject group. Also, dividing Alzheimer's disease patients into 3 groups (those that do not carry APO E4, those carrying APO E4 as a hetero zygote, those carrying APOE4 as a homo zygote) demonstrated that the disease develops at younger ages as the prevalence of APO E4 increases.

Also, active studies are ongoing for risk factors other than APO E4, the genetic polymorphism of the VLDL receptor, an apolipoprotein E receptor.

With respect to obesity, the ob protein has recently been identified (Nature, 372, p. 425, 1994) and it has been reported that there is a feedback circuit in which obesity promotes the expression and secretion of the ob protein in fat cells, which in turn stimulates the hypothalamic satiety center to suppress food intake and to increase energy consumption. A study with a hereditary obesity model mouse, known as the ob/ob mouse, is now being conducted to correlate the ob protein (leptin) to human obesity. In addition, the relationship between obesity and the β3 adrenaline receptor is also drawing attention. In other words, obesity can develop due to decreased energy consumption, as well as excessive energy intake.

In humans, as well as in rats and mice, excessive food intake and obesity are thought to potentially stimulate sympathetic nervous system β3 receptor activity to increase energy consumption and cause thermogenesis, especially in brown fat cells etc. This pathway may also be viewed as a feedback mechanism for suppression of the progression of obesity. In cases where such feedback route fails to function due to a congenital anomaly in the β3 receptor, energy consumption is not promoted even by obesity or excessive food intake, resulting in further obesity. In fact, a mutation of the β3 adrenalin receptor by replacement of the 64th Trp residue with an Arg residue has been reported in the Pima Indian tribe which is characterized by high prevalence of obesity and diabetes mellitus [N. Engl. J. Med., 333, p. 343 (1995)]. The same mutation has been identified in people of Caucasian and Finnish descent. This gene mutation, unlike the known gene variations in diabetes mellitus (mutations in the genes for insulin, insulin receptor, glucokinase and mitochondria), is observed at very high frequencies of about 8% in Caucasians and 31% in Pima Indians. Accordingly, it is now recognized that obesity may be a disease caused at the gene level rather than a habitual or cultural problem.

Non-insulin-dependent diabetes mellitus (NIDDM) is known to develop due to an interaction of both genetic and environmental factors. Its pathogenesis is associated with multiple-factor inheritance involving not a single gene but a number of genes. Although such genetic factors remain to be clarified, some candidate genes are known. The elucidation of such risk factors for diabetes mellitus pathogenesis, will assist in early diagnosis, early treatment and prevention of onset of diabetes mellitus and would reduce medical expenses required in case of aggravation of diabetes mellitus with complications, and is therefore strongly demanded desired to reduce medical costs due to this illness.

Candidate genes that may be profoundly involved in NIDDM include genes that cause insulin secretion anomaly, and genes that cause insulin resistance. Of these genes, those believed to result in anomaly in insulin secretion involve genes for of insulin glucokinase and for mitochondria. Genes involved in skeletal muscle or liver insulin sensitivity reduction (insulin resistance) that have been identified so far include genes for insulin receptors and for glucokinase, an enzyme is involved in hepatic saccharide uptake. Abnormalities in these genes may be involved in NIDDM.

The causal relationship between glucokinase abnormalities and MODY (maturity-onset diabetes in the young) disease has been extensively studied. Glucokinase is a rate-limiting enzyme for glucose metabolism of pancreatic B cells and hepatocytes. The candidate genes for this enzyme are very interesting in that the pathologic characteristics of NIDDM, i.e., abnormal secretion of insulin and insulin resistance, may be explained by a single gene abnormality. MODY is also an early onset subtype of NIDDM with autosomal dominant inheritance that develops at less than 25 years of age, accompanied by glucokinase mutation, which is assumed to be the cause of the disease.

Separately, the relationship between insulin receptor abnormalities and NIDDM has been studied. The insulin receptor consists of a total of 1,355 amino acid residues; a point mutation has been proven to cause insulin resistance. Insulin receptor abnormalities appear in co-dominant inheritance, in which a phenotype appears even in the case of hetero zygotes, and a slightly stronger phenotype appears in the case of homo zygotes.

In addition to these studies, the association of mitochondrial gene abnormalities and NIDDM has also been studied. These 3 types of gene abnormalities i.e. abnormalities in the genes of a glucokinase, insulin receptor and mitochondria, can be viewed as cause of diabetes mellitus because each causes a particular subtype of diabetes mellitus by a single gene abnormality. However, the β3 adrenaline receptor, described above, cannot be recognized as an etiologic gene, even though it serves as a strong pathogenetic factor for NIDDM. It appears that the genes for the β3 adrenaline receptor along with some other genes that are involved in the pathogenesis of obesity of NIDDM are the under influence of environmental factors.

It is well known that environmental factors are involved in the pathogenesis and progression of hypertension. However, in recent years, genetic predispositions to hypertension have been elucidated. With regard to essential hypertension, which reportedly accounts for more than 90% of hypertensive cases, however, more time is necessary to completely elucidate the pathology, although genetic factors associated with its pathogenesis have been demonstrated.

Human hypertension is characterized by multiple-factor inheritance, incomplete penetrance and strong influence from environmental factors. Currently, approaches used for genetic analysis using human populations include (1) linkage analysis using the DNA of a family member, in the case of evident familial diseases, (2) the affected sib-pair method, which uses the DNA of an affected sib-pair, and (3) the association study method. Of these methods, linkage analysis is very useful in the identification of causative genes for special forms of hypertension which show a strong genetic background, while the affected sib-pair method and the association study method are used to analyze genetic risk factors in essential hypertension.

Essential hypertension, which presumably accounts for most hypertensive patients, has not undergone extensive linkage analysis, because DNA collection is difficult over 3 generations due partially to slow pathogenesis. In these circumstances, the affected sib-pair method has recently yielded remarkable results.

In 1992, Jeunemaitre et al. identified 15 variations on the angiotensinogen gene, and using the affected sib-pair method for hypertensive sib-pairs in the State of Utah, USA and in Paris, France, demonstrated significant correlation of the M235T (single-base substitution of the 235th codon from Met to Thr) variation in exon 2 and hypertension (Cell, 71, p. 169, 1992).

This correlation was stronger in severe hypertension cases; the mutation was shown to increase blood angiotensinogen concentrations in humans of the TT genotype, as well as the ACD/DD polymorphic genotype.

A report on the correlation of a point mutation (A1166C) in the AII1 type receptor gene and hypertension (Hypertension, 24, p. 63, 1994), reports on blood pressure reduction in AT1-R gene knock-out mice and blood pressure elevation in AII2 type receptor gene knock-out mice (Proc. Natl. Acad. Sci. USA, 92, p. 3521, 1995; Nature, 377, p. 744, 1995) and other published data suggest the genetic involvement of AII aberrant receptor genes in blood pressure regulation in some way.

Moreover, Zee et al. reported that an abnormality in the low-density lipoprotein receptor gene is associated with obesity in hypertensive patients (Biochem. Biophys. Res. Commun., 189, p. 965, 1992), and Hagihara et al. reported the involvement of the GJP (gap junction protein) gene locus in SHR body weight (Hypertens. Res., 18, p. 63, 1995).

Although there have been steady advances in the research into hypertension at the gene level, as stated above, it appears that considerable time is necessary to further clarify the essential etiologic factors therefor because long-term extensive genetic immunological studies must be done.

Factors involved in heart diseases have also been studied at the gene level. A group of researchers at Bringham and Woman's Hospital, Boston, USA reported that they localized 2 genes that suppress myocardial infarction and 1 gene that increases the risk of myocardial infarction (Nature Genetics, 13, p. 429, 1996).

Although many malignant tumors are thought to have a hereditary, hereditary component tumors showing what is called autosomal dominant inheritance develop in a smaller number of cases. There are still only a small number of such tumors that have had their association with chromosomes or genes clarified.

The great majority of cancers are diseases to which mendelian genetics, which is based on variations in single genes, is not applicable, or which do not enable the candidate gene approach for exploration of their cause at the gene level. This does not mean, however, that common cancers are not always solitary or hereditary. Genetic etiologic studies have shown that the relatives of a particular cancer patient have an increased risk of developing of similar tumors. It is therefore reasonable from the viewpoint of multiple-factor genetics to assume that a number of environmental factors and genetic factors serve as prerequisites for oncogenesis.

As stated above, multiple-factor genetic models are applicable to cancers; in fact, cancers are gene-associated diseases. It has been demonstrated that oncogenesis occurs in multiple steps as a result of synergistic accumulation of a number of abnormalities in cancer genes and cancer suppressor genes.

For colorectal cancer, the multiple-step oncogenesis process has been studied extensively. It is assumed that variations in cancer genes such as Ras activate cell proliferation. In contrast an abnormality in the tumor suppressor gene p53 inactivates the functioning of normal genes, resulting in the failure to control cell proliferation.

A group of researchers at Columbia University, USA has recently succeeded in cloning PTI-1, a gene that promotes cell malignancy from prostatic hypertrophy to prostatic cancer. PTI-1 serves as a switch from benign adenomas to malignant tumors.

This factor is a gene that regulates the translation process to affect translation. It is a 46 kd protein homologous to the polypeptide chain elongation factor EF1a (50 kd). PTI-1 is thought to result from point mutation and deletion in EF1. The study by the same group confirmed PTI-1 expression in 15 of their 16 patients with progressive prostatic cancer.

Although efforts to discover genes for the etiologic or risk factors for various diseases have steadily yielded good results, much information remains to be obtained before the pathogenetic profiles are fully elucidated.

The step of determining the cause of pathogenesis and functional analysis of genetic information subsequent to determining the sequence and function of a disease-associated gene, is an important task for judging whether the abnormality in the gene is an outcome of disease progression or a pathogenetic cause. As used herein a gene is considered the "cause" of a disease if an aberation (i.e. mutation) in that gene is correlated with onset of diseases.

However, the information obtained in the previous step (discovery of disease-associated genes) is nothing more than gene sequence information. There are currently almost no methods to accurately deduce the function of the gene or gene product from this information.

The knock-out transgenic mouse preparation technology may now be the most feasible approach. In the prior art technology, however, unexpected serious damage to the living body by gene manipulation often results in still-born delivery or neonatal death, which in turn hampers the advance of the study. The knock-out transgenic mouse preparation technology is expected to make major contributions to the analysis of gene function, provided that there will be further advances.

Although bioinformatics has potential for the clarification of gene function, it is currently in the initial stage of compiling basic information. The speed of gene sequencing has been remarkably increased; the human cDNA sequence has already been disclosed to a fair degree. During the next several years, advances in the analysis of gene function by a large number of universities and genome venture companies are expected, although their efforts will be accompanied by many difficulties. Such quantitative increase in basic information is likely to have synergistic effects to dramatically accelerate the analysis of gene function by bioinformatics after an unknown time point as a powerful tool of functional analysis of genes.

After analysis of the sequence and function of a disease-associated gene, resulting in the identification of the etiologic gene, development of a new pharmaceutical will begin as the third step based on the information obtained from the previous two steps. However, there is no well-established technology for such drug creation.

### Summary of Invention

Against this background, there is a need for the establishment of a new technology for identifying and/or creating drugs based on information obtained from analysis of structural genes associated with human diseases that elucidates the causal relationship between a gene polymorphism and a disease. The present invention provides such a new method of pharmaceutical development, and a new use for a substance capable of operating an aberrant gene product on the basis thereof.

After extensive investigation in an attempt to resolve the above problems, the present inventor succeeded in cloning the genes for aberrant gene products (aberrant receptors etc.) from cDNA libraries prepared from human cells or commercially available cDNA libraries derived from human cells, and developed the present invention using cells transformed with those genes.

The present invention relates to a method of creating drugs that enables an approach from the cause of pathogenesis, unlike conventional new drug development approach, which is based on the results of pathogenesis, such as the mechanism of pathogenesis and drug action mechanism. Use of the drug creation technology of the present invention enables a drug creation approach totally differing from the conventional approach. The present invention has a major impact on the development of therapeutic drugs for diseases associated with the brain/central nervous system, wherein the naturally occurring ligands are of low molecular weights.

More specifically, the present invention provides:
(1) a pharmaceutical composition comprising a substance capable of causing an aberrant gene product to function in a manner similar to the wild-type gene product, that is, operating, and a pharmaceutically acceptable carrier,
(2) the pharmaceutical composition according to paragraph (1), the substance is an agonist or an antagonist of aberrant gene product,
(3) the pharmaceutical composition according to paragraph (1), wherein the aberrant gene product is an aberrant receptor, an aberrant channel, an aberrant signal or an aberrant enzyme,
(4) the pharmaceutical composition according to paragraph (1), which contains a substance capable of operating an aberrant operator and which is for prophylaxis and/or treatment of a disease caused by an aberrant receptor,
(5) the pharmaceutical composition according to paragraph (4), wherein the disease caused by an aberrant receptor is a disease caused by substantial reduction in the activity of the signal transduction system of the cell having the aberrant receptor,
(6) the pharmaceutical composition according to paragraph (4), wherein the disease caused by an aberrant receptor is a disease caused by substantial absence of the action of a natural ligand on the transfer system of the cell having the aberrant receptor,
(7) the pharmaceutical composition according to paragraph (4), wherein the disease caused by an aberrant receptor is a disease caused by substantial reduction in the affinity of a natural ligand for the aberrant receptor,
(8) the pharmaceutical composition according to paragraph (6), wherein the signal transduction system is a signal transduction system based on the change in intracellular concentration of a responding substance resulting from the binding of a natural ligand and a receptor,
(9) the pharmaceutical composition according to paragraph (6), wherein the responding substance is cAMP, inositol phosphate or calcium ion,
(10) the pharmaceutical composition according to paragraph (4), wherein the substance capable of operating an aberrant receptor is a substance that operates the normal receptor,
(11) the pharmaceutical composition according to paragraph (4), wherein the substance capable of operating an aberrant receptor is a substance that does not operate the normal receptor,
(12) a use for a substance capable of operating an aberrant gene product, which is for treatment of a disease caused by said aberrant gene product,
(13) the use according to paragraph (12), wherein the aberrant gene product is an aberrant receptor,
(14) a method of screening a substance capable of operating an aberrant gene product, which comprises bringing an aberrant gene product into contact with a subject substance and assaying the operation activity of said substance on said product,
(15) the screening method according to paragraph (14), wherein the aberrant gene product is an aberrant receptor,
(16) a method of screening a substance for treatment of a disease caused by an aberrant gene product, which comprises bringing an aberrant gene product into contact with a subject substance and assaying the operation activity of said substance on said product,
(17) a method of screening a drug for substantially operating the signal transduction system of a cell having an aberrant receptor of a mammal suffering from a disease caused by the aberrant receptor, which comprises bringing the aberrant receptor into contact with a subject substance and assaying the operation activity of said substance on said receptor,
(18) the screening method according to paragraph (16), wherein the aberrant receptor is an aberrant receptor prepared by expressing in a cell the gene encoding the aberrant receptor,
(19) the screening method according to paragraph (16), wherein the gene encoding the aberrant receptor is an aberrant receptor-encoding gene specified by comparative analysis of a gene prepared from a cell of a mammal suffering from a disease caused by the aberrant receptor, and a gene prepared from a cell of a mammal of the same species that does not carry the aberrant receptor,
(20) a method of preparing a drug for treatment of a disease caused by an aberrant gene product, which comprises bringing the aberrant gene product into contact with a subject substance, assaying the operation activity of said substance on said product and preparing a substance judged to substantially operate the signal transduction system of a cell having the aberrant gene product,
(21) a method of preparing a substance for treatment of a disease caused by an aberrant receptor, which comprises bringing the aberrant receptor into contact with a subject substance, assaying the operation activity of said substance on the aberrant receptor and preparing a substance judged to substantially operate the transfer system of a cell having the aberrant receptor,
(22) the method according to paragraph (21), wherein the aberrant receptor is an aberrant receptor prepared by expressing in a cell the gene encoding the aberrant receptor,
(23) the method according to paragraph (22), wherein the gene encoding the aberrant receptor is an aberrant receptor-encoding gene specified by comparative analysis of a gene prepared from a cell of a mammal suffering from a disease caused by the aberrant receptor, and a gene prepared from a cell of a mammal of the same species that does not carry the aberrant receptor,
(24) a use for an aberrant gene product obtained by expressing in a cell the gene encoding the aberrant gene product, which is for screening for a substance for treatment of a disease caused by said product,
(25) the use according to paragraph (24), wherein the aberrant gene product is an aberrant receptor,
(26) the use for an aberrant gene product obtained by expressing in a cell the gene encoding the aberrant gene product, which is for screening for a substance that normally operates said product, and
(27) the use according to the paragraph (26), wherein the aberrant gene product is an aberrant receptor.

### Detailed Description of The Invention

The term "aberrant gene product" as used herein includes, but is not limited to aberrant receptors, aberrant channels, aberrant signal and aberrant enzymes. The term "aberrant" above means a mutant in the structural gene corresponding to a gene product, particularly a mutant that has occurred naturally in vivo, which mutant can cause a disease due to the fact that the reactivity of a substance that operates the normal gene product (e.g., the ligand that operates the normal receptor, such as a natural ligand present in vivo) differs from the reactivity of said substance which the normal gene product. Also, it is preferable that the aberrant gene product and the normal gene product are capable of similar of function, i.e., after a substance against the aberrant gene product results in the aberrant gene product acting normally, the aberrant gene product should exhibit a response (e.g., change in intracellular concentration of responding substance in the signal transduction system of cells having the normal receptor) similar to that exhibited by the normal gene product after being operated by an operating substance thereagainst.

Diseases caused by aberrant gene products include, but are not limited to Alzheimer's disease (e.g., familial Alzheimer's disease, solitary Alzheimer's disease), schizophrenia, depression, hypertension (e.g., essential hypertension), obesity, diabetes mellitus (e.g., non-insulin-dependent diabetes mellitus), heart diseases (e.g., myocardial infarction), cancers (e.g., colorectal cancer, prostatic cancer), rheumatism, allergic diseases (e.g., atopy) and arteriosclerosis, with preference given to Alzheimer's disease, schizophrenia, depression, hypertension, obesity, diabetes mellitus, cancers etc.

The term "aberrant receptor" as used herein is understood to include receptors with a mutation in the structural gene thereof in vivo, resulting in substantially changed affinity for substances such as the natural ligand (e.g., reduction, enhancement etc., preferably reduction), particularly receptors that cause a disease due to the substantial change in the affinity of said natural ligand. The term "substantial change" as used above means a change to the extent that a disease can be caused when the affinity of the natural ligand for the normal and aberrant receptors are compared, and may be any change, whether significant or insignificant, as long as it is capable of causing a disease. Also, it is preferable that the aberrant receptor and the normal receptor be capable of similar function, i.e., after an operating substance against the aberrant receptor results in the aberrant receptor acting normally, the aberrant receptor should exhibit a response (e.g., change in intracellular concentration of responding substance in the signal transduction system of cells having the normal receptor) similar to that product by the normal receptor after being operated by a natural ligand thereagainst. It is preferable that the signal transduction system of cells having the aberrant receptor be identical to that of cells having the normal receptor. In other words, if the affinity of the natural ligand has been substantially decreased due to a mutation in the receptor gene, even if the cells having the aberrant receptor have the same signal transduction system as that of cells having the normal receptor, the signal transduction system of the cells having the aberrant receptor substantially will fail to function, which can cause a disease. However, according to the present methods, the use of an agonist for the aberrant receptor that binds to the aberrant receptor and which is capable of operating the signal transduction system of the cells having the aberrant receptor would enable the effective prevention or treatment of such a disease.

The disease caused by an aberrant receptor may be any disease, as long as it is caused by a substantial change in the affinity of the natural ligand. Such diseases include diseases caused by substantial reduction in the affinity of the natural ligand for the receptor, diseases caused by substantial failure of the natural ligand to operate the signal transduction system of the cells having the aberrant receptor, diseases caused by substantial reduction in the activity of the transfer system of the cells having the aberrant receptor and diseases caused by aberrant signaling or excessive signaling in the signal transduction system of the cells having the aberrant receptor. More specifically, such diseases include Alzheimer's disease (e.g., familial Alzheimer's disease, solitary Alzheimer's disease), schizophrenia, depression, hypertension (e.g., essential hypertension), obesity, diabetes mellitus (e.g., non-insulin-dependent diabetes mellitus), heart diseases (e.g., myocardial infarction), cancers (e.g., colorectal cancer, prostatic cancer), rheumatism, allergic diseases (e.g., atopy) and arteriosclerosis, with preference given to Alzheimer's disease, schizophrenia, depression, hypertension, obesity, diabetes mellitus, cancers etc.

Substances capable of operating an aberrant receptor include agonist and antagonists to the aberrant receptor and are termed "aberrant receptor agonists" and "aberrant receptor antagonists", respectively. These substances are used as appropriate for the target disease. For example, in diseases caused by substantial reduction in the affinity of the natural ligand for the receptor, diseases caused by substantial failure of the natural ligand to operate the signal transduction system of the cells having the aberrant receptor, diseases caused by substantial reduction in the activity of the transfer system of the cells having the aberrant receptor, and other such diseases, aberrant receptor agonists are preferably used. In diseases caused by abnormal signaling or excessive signaling in the signal transduction system of the cells having the aberrant receptor, and other such diseases, aberrant receptor antagonists are preferably used. Also, the aberrant receptor-operating substance may be a substance that operates the normal receptor or a substance that does not operate the normal receptor, selected depending on the target disease, with preference given to a substance that substantially fails to operate the normal receptor.

Such signal transduction systems include those based on a change in intracellular concentrations of responding substances (e.g., cAMP, inositol phosphate, calcium ion) formed by the binding of a natural ligand and a receptor.

The term "aberrant channel" as used herein is understood to include channels with a mutation in the structural gene thereof, resulting in substantially changed affinity of substances (e.g. blockers, openers) that operate the normal channel, with preference given to channels that cause a disease due to the substantial change in the affinity of said operator. Also, it is preferable that the aberrant channel and the normal channel be capable of similar of function, i.e., after an operating substance against the aberrant channel results in the aberrant channel acting normally, the aberrant channel should exhibit a response (e.g., gate closure or opening) similar to that exhibited by the normal channel after being operated by an operating substance thereagainst. Diseases caused by aberrant enzymes include Alzheimer's disease (e.g., familial Alzheimer's disease, solitary Alzheimer's disease), schizophrenia, depression, hypertension (e.g., essential hypertension), obesity, diabetes mellitus (e.g., non-insulin-dependent diabetes mellitus), heart diseases (e.g., myocardial infarction), cancers (e.g., colorectal cancer, prostatic cancer), rheumatism, allergic diseases (e.g., atopy) and arteriosclerosis, with preference given to Alzheimer's disease, schizophrenia, depression, hypertension, obesity, diabetes mellitus, cancers etc.

The term "aberrant signal" as used herein is understood to include signals with a mutation in the structural gene thereof, resulting in substantially changed affinity of substances that operate the normal signal (e.g., substances that promote or inhibit the signal transduction by signals), with preference given to signals that cause a disease due to the substantial change in the affinity of said operating substance. Also, it is preferable that the aberrant signal and the normal signal be capable of similar of function, i.e., after an operating substance against the aberrant signal is used on the aberrant signal, the aberrant signal exhibits a response similar to that exhibited (e.g., transportation of ions, nutrients etc.) by the normal signal after being operated by an operating substance thereagainst.

Diseases caused by aberrant signals include Alzheimer's disease (e.g., familial Alzheimer's disease, solitary Alzheimer's disease), schizophrenia, depression, hypertension (e.g., essential hypertension), obesity, diabetes mellitus (e.g., non-insulin-dependent diabetes mellitus), heart diseases (e.g., myocardial infarction), cancers (e.g., colorectal cancer, prostatic cancer), rheumatism, allergic diseases (e.g., atopy) and arteriosclerosis, with preference given to Alzheimer's disease, schizophrenia, depression, hypertension, obesity, diabetes mellitus, cancers etc.

The term "aberrant enzyme" as used herein is understood to include enzymes with a mutation in the structural gene thereof, resulting in substantially changed affinity to substances (e.g., activators, inhibitors) that operate the normal enzyme, with preference given to enzymes that cause a disease due to the substantial change in the affinity of said operating substance. Also, it is preferable that the aberrant enzyme and the normal enzyme be capable of similar of function, i.e., after an operating substance against the aberrant enzyme is used on the aberrant enzyme, the aberrant enzyme should exhibit a response (e.g., substrate protein activation) similar to that exhibited by the normal enzyme after being operated by an operating substance thereagainst.

Diseases caused by aberrant enzymes include Alzheimer's disease (e.g., familial Alzheimer's disease, solitary Alzheimer's disease), schizophrenia, depression, hypertension (e.g., essential hypertension), obesity, diabetes mellitus (e.g., non-insulin-dependent diabetes mellitus), heart diseases (e.g., myocardial infarction), cancers (e.g., colorectal cancer, prostatic cancer), rheumatism, allergic diseases (e.g., atopy) and arteriosclerosis, with preference given to Alzheimer's disease, schizophrenia, depression, hypertension, obesity, diabetes mellitus, cancers etc.

Identification of the gene encoding an aberrant gene product (e.g., aberrant receptor) can be achieved by comparing genes prepared from a cell of a mammal (e.g., rats, rabbits, sheep, pigs, bovines, cats, dogs, monkeys, humans, preferably humans) suffering from a disease caused by the aberrant gene product, and genes prepared from a cell of a mammal of the same species that does not have the disease and therefore, does not carry the aberrant gene product.

An aberrant gene identified as a disease-causing gene by analyzing of the sequence and function of the gene (e.g., genes encoding aberrant gene products derived from human cells, preferably genes encoding aberrant receptors derived from human cells, etc.) can, for example, be prepared by the method described below.

First, the RNA encoding an aberrant gene product is prepared from a cell of an animal species from which said gene product is derived (e.g., human cells), preferably cells of animals suffering from a disease caused by the aberrant gene product. Available methods of preparing RNA from such materials include the guanidine thiocyanate method [J.W. Chirgwin et al., Biochemistry, Vol. 18, p. 5,294 (1979)].

After an oligo-dT primer or random oligonucleotide is added to the RNA thus obtained, cDNA can be synthesized in the presence of reverse transcriptase. PCR can be carried out in the presence of a sense primer and antisense primer, both for amplification of the cDNA of an aberrant gene product from a standard cDNA preparation obtained on the basis of a published sequence or analytically identified sequence of said product, PCR can be carried out, as directed in the instruction manual for a commercially available kit (e.g., produced by Cetus/Perkin-Elmer). The amplified cDNA can be separated by a commonly known method, e.g., agarose electrophoresis, and then recovered from the gel. The base sequence of this cDNA can be determined by the dideoxynucleotide synthetic chain termination method [T. Messing et al., Nucl. Acids Res., Vol. 9, p. 309 (1981)].

The plasmid having the cloned cDNA can be used as such, or after being cleaved with an appropriate restriction enzyme and inserted into another vector as desired.

Any vector can be used, as long as it enables replication in the host. When the host is a bacterium of the genus *Escherichia (*e.g*. Escherichia coli),* such vectors include *Escherichiacoli*-derived plasmids, e.g., pBR322 [F. Bolivar et al., Gene, Vol. 2, p. 95 (1979)], pBR325, pUC12 and pUC13. When the host is a yeast, such vectors include yeast-derived plasmids, e.g., pSH19 [S. Harashima et al., Mol. Cell. Biol., Vol. 4, p. 771, (1984)] and pSH19-1 (European Patent Application Publication EP-A-0235430). When the host is an animal cell, such vectors include, for example, pSV2-X [R.C. Mulligan and P. Berg, Proc. Natl. Acad. Sci. USA, Vol. 78, p. 2072 (1981)], which results from SV40 ori insertion into pBR322, and pcD-X [H. Okayama and P. Berg, Mol. Cell. Biol., Vol. 3, p. 280 (1983)]. When the host is an insect cell, such vectors include, for example, the Baculovirus transfer vectors pVL1392 and pVL1393 [manual (MAXBAC^{tm} Baculovirus expression system, Manual version 1.4) supplied by the manufacturer (Invitrogen Corporation, CA, USA)].

The cloned cDNA may have the translation initiation codon (ATG) at the 5'-terminal thereof and the translation termination codon (TAG, TGA or TAA) at the 3'-terminal thereof. To enhance the expression of said cDNA, it is preferable that the promoter sequence be joined upstream and operably linked thereto.

Any promoter can be used for the present invention, as long as it is appropriate for the host used to express the desired gene. When the host is *Escherichia coli,* such promoters include the T7 promoter, trp promoter, tac promoter, lac promoter and λPL promoter, with preference given to the T7 promoter. When the host is a yeast, such promoters include the GAPDH promoter, PGK promoter, PHO5 promoter and ADH promoter, with preference given to the GAPDH promoter. When the host is an animal cell, such promoters include the SV40-derived promoter, retrovirus promoter and human cytomegalovirus promoter. When the host is an insect cell, such promoters include the polyhedrin promoter of nuclear polyhedrosis virus.

A promoter can be prepared from the corresponding gene. It can also be chemically synthesized. The expression vector may contain a signal sequence, pre-pro sequence etc., which may be selected from known signals, as long as they function in the host.

Using the thus-constructed DNA-containing recombinant expression plasmid, a transformant is produced

Hosts include, for example, bacteria of the genus *Escherichia,* yeasts, animal cells and insect cells. Bacteria of the genus *Escherichia* include *Escherichia coli* K12 DH1 [B. Low, Proc. Natl. Acad. Sci. USA, Vol. 60, p. 160 (1968)], C600 [R.K. Appleyard, Genetics, Vol. 39, p. 440 (1954)], MM294 [K. Backman et al., Proc. Natl. Acad. Sci. USA, Vol. 73, p. 4,174 (1976)] and N4830 [M.E. Gottesman et al., J. Mol. Biol., Vol. 140, p. 57 (1980)]. Yeasts include, for example, *Saccharomyces cerevisiae* AH22R⁻ [A. Miyanohara et al., Proc. Natl. Acad. Sci. USA, Vol. 80, p. 1 (1983)], NA87-11A, DKD-5D, NA74-3A and NA74-3Aρ⁻ [Y. Kaisho et al., Yeast, Vol. 5, p. 91 (1989)] and *Saccharomyces pombe* ATCC38399 (h-leul-32) and TH168 (h90 ade6-M210 ural leul) [M. Kishida and C. Shimada, Current Genetics, Vol. 10, p. 443 (1986)]. Animal cells include, for example, simian COS-7 cells, simian Vero cells, Chinese hamster ovarian (CHO) cells, mouse L cells, human FL cells, which are all adhesion cells, and mouse myeloma cells (Sp2/0 cells etc.), mouse YAC-1 cells, mouse MethA cells, mouse P388 cells and mouse EL-4 cells, which are all suspension cells. Insect cells include Sf9 cells.

Bacteria of the genus *Escherichia* can be transformed in accordance with the method described by T. Maniatis et al. [Molecular Cloning, Cold Spring Harbor Laboratory, p. 249 (1982)], for instance. Yeasts can be transformed in accordance with the method described by A. Hinnen (Proc. Natl. Acad. Sci. USA, Vol. 75, p. 1929 (1978), for instance. Animal cells can be transformed in accordance with the method described by M. Wigler et al. in Cell, Vol. 14, p. 725 (1978), for instance. Insect cells can be transformed in accordance with the manual (MAXBAC^{tm} Baculovirus expression system, Manual version 1.4) supplied by the manufacturer (Invitrogen Corporation).

The transformant thus obtained is cultured by a commonly known method.

Media preferably used to cultivate a transformant whose host is a bacterium of the genus *Escherichia* include, for example, the M9 medium containing glucose and casamino acid [J.H. Miller, Experiments in Molecular Genetics, p. 431, Cold Spring Harbor Laboratory (1972)]. To increase promoter efficiency as necessary, a chemical agent such as isopropyl thiogalactoside (IPTG) or indolyl-3-acrylic acid may be added. Cultivation is normally carried out at about 15 to 43°C for about 3 to 24 hours, with aeration and/or stirring as necessary.

Media for cultivating a transformant whose host is a yeast include, for example, Burkholder's minimal medium [K.L. Bostian et al., Proc. Natl. Acad. Sci. USA, Vol. 77, p. 4504 (1980). It is preferable that the medium be adjusted to pH about 5 to 8. Cultivation is normally carried out at about 20 to 35°C for about 24 to 72 hours, with aeration and/or stirring as necessary.

Media for cultivating a transformant whose host is an animal cell include, for example, MEM medium containing about 5 to 20% fetal bovine serum [H. Eagle, Science, Vol. 130, p. 432 (1959)], DMEM medium [R. Dulbecco and G. Freeman, Virology, Vol. 8, p. 396 (1959)], RPMI-1640 medium [G.E. More et al., Journal of the American Medical Association, Vol. 199, p. 519 (1967)], 199 medium [J.F. Morgan et al., Proceedings of the Society for Experimental Biology and Medicine, Vol. 73, p. 1 (1950)] and ASF104 medium (Ajinomoto). Cultivation is normally carried out at about 30 to 40°C for about 15 to 60 hours, with aeration and/or stirring as necessary.

Media for cultivating a transformant whose host is an insect cell include, for example, TNM-FH medium [W.F. Hink et al., Nature, Vol. 226, p. 466 (1990). Cultivation is normally carried out at about 15 to 30°C for about 24 to 72 hours, with aeration and/or stirring as necessary.

According to the present invention, an expression product (aberrant gene product such as an aberrant receptor) can be isolated by appropriate combinations of commonly known methods of separation and purification. Such known methods of separation and purification include those based on solubility differences, such as salting-out and solvent precipitation; those based mainly on molecular weight differences, such as dialysis, ultrafiltration, gel filtration and SDS-polyacrylamide gel electrophoresis (SDS-PAGE); those based on charge differences, such as ion exchange chromatography; those based on specific affinity, such as affinity chromatography; those based on hydrophobicity differences, such as reverse-phase high performance liquid chromatography; and those based on isoelectric point differences, such as isoelectric focusing.

By expressing an aberrant genetic product by gene engineering technology using *Escherichia coli,* a cultured animal cell, a cultured insect cell etc., the desired product can be produced at high purity and in large amounts.

When the thus-obtained aberrant gene product is in free form, it can be converted to a salt by known methods or a method based thereon. When the aberrant gene product is obtained in salt form, it can be converted to free form or another salt by a commonly known method or a method based thereon.

Also, it is possible to express an aberrant gene product (e.g., aberrant receptor of humans etc.) in a eukaryotic cell using the product's cDNA, and use the aberrant gene product obtained to search for a compound that operates it. Alternatively, by using the gene encoding a specified aberrant gene product as a DNA probe, the mRNA content in the aberrant gene product expressed in vivo can be determined by the Northern blotting method. Moreover, it is possible to prepare an antibody against said aberrant gene product, and quantify said product in vivo by in situ hybridization.

This aberrant gene product can be used for screening for a substance capable of operating an aberrant gene product, screening for drugs for treatment of diseases caused by aberrant gene products (e.g., screening for drugs for operating the transfer system of a cell having an aberrant receptor of a mammal suffering from a disease caused by the aberrant receptor) and for other purposes, and is especially useful for screening for agonists or antagonists for disease-associated aberrant receptors in warm-blooded animals such as humans.

The above screening is hereinafter described more specifically.

The above-described aberrant gene product is useful in searching for an operating substance thereof. In other words, it enables the provision of a screening method for a substance capable of operating an aberrant gene product, wherein said product is brought into contact with a subject substance to determine the effect of the subject substance on the aberrant gene product.

Useful subject substance include known ligands, synthetic compounds, peptides, proteins etc., as well as tissue extracts and cell culture supernatants of warm-blooded mammals (e.g., mice, rats, pigs, bovines, sheep, monkeys, humans). For example, such a tissue extract, cell culture supernatant, or the like, may be added to the above-described aberrant gene product, followed by fractionation with monitoring of operation activity etc., to finally yield a single operating substance.

Specifically, the screening method is exemplified by the method in which substances (e.g., peptides, proteins, non-peptide compounds, synthetic compounds, fermentation products) having operational activity on said product are tested by constructing an expression system for an aberrant gene product (e.g., aberrant receptor) and adding the subject substance to the expression system. In screening for an operating substance for an aberrant receptor, in particular, it is preferable that an aberrant receptor prepared by expressing in a cell the gene encoding the aberrant receptor be used.

More specifically, the present invention provides a screening method for a substance capable of operating an aberrant gene product, characterized in that:
① the amount of labeled subject substance bound to an aberrant gene product (e.g., aberrant receptor) is determined when the labeled substance is brought into contact with said product,
② the amount of labeled subject substance bound to cells containing an aberrant gene product (e.g., aberrant receptor) or a membrane fraction of said cells is determined when the labeled subject substance is brought into contact with said cells or the membrane fraction thereof,
③ the amount of labeled subject substance bound to an aberrant gene product (e.g., aberrant receptor) is determined when the labeled subject substance is brought into contact with said product expressed on the cell membrane of a transformant containing DNA encoding the product by culturing said transformant,
④ a cell-stimulating activity (e.g., growth promotion, promotion or suppression of intracellular protein phosphorylation) via an aberrant gene product (e.g., aberrant receptor) is used to assay the operation activity of a subject substance when said substance is brought into contact with cells containing said product, and
⑤ a cell-stimulating activity (e.g., growth promotion, promotion or suppression of intracellular protein phosphorylation) via an aberrant gene product (e.g., aberrant receptor) is used to assay the operation activity of a subject substance when said substance is brought into contact with the aberrant gene product as expressed on the cell membrane of a transformant containing DNA encoding the abnormal gene product by culturing said transformant.

Labeled subject substances include substances labeled with [³H], [¹²⁵I], [¹⁴C], [¹³⁵S] or other radioisotopes.

Example procedures for screening for a substance capable of operating an aberrant gene product are hereinafter described.

First, a standard aberrant receptor preparation is prepared by suspending cells containing the aberrant receptor or the membrane fraction thereof in an appropriate buffer. Any buffer can be used, as long as it does not interfere with subject substance-aberrant receptor binding. Such buffers include phosphate buffers and Tris-HCl buffers of pH about 4-10 (preferably pH 6-8). For the purpose of reducing non-specific binding, surfactants such as CHAPS, Tween-80 (trade name) (Kao-Atlas), digitonin and deoxycholate, and various proteins such as bovine serum albumin and gelatin, may be added to the buffer. Also, for the purpose of inhibiting degradation of the receptor and subject substance by protease, protease inhibitors such as PMSF (phenylmethanesulfonyl fluoride), leupeptin, E-64 (produced by Peptide Institute, Inc.) and pepstatin may be added. To 0.01 ml to 10 ml of said receptor solution, a subject substance labeled with a given amount (5,000 cpm to 500,000 cpm) of [³H], [¹²⁵I], [¹⁴C] or the like is added. To determine the amount of non-specific binding (NSB), a reaction tube containing an unlabeled subject substance in excess is also provided. Reaction is carried out at 0°C to 50°C, preferably 4°C to 37°C for 20 minutes to 24 hours, preferably 30 minutes to 3 hours. After completion of the reaction, the reaction mixture is filtered through glass fiber filter paper etc. and washed with an appropriate amount of the same buffer, after which the residual radioactivity in the glass fiber filter is measured using a liquid scintillation counter or γ-counter. A subject substance yielding the radioactivity above 0 cpm in count (B - NSB) obtained by subtracting nonspecific binding (NSB) from total binding (B) may be selected as a ligand for the aberrant receptor.

To assess the operation activity of a subject substance on an aberrant receptor, cells containing the aberrant receptor are first cultured on multiwell plates etc. Prior to agonist assay, the medium is replaced with fresh medium or an appropriate buffer that is non-toxic to the cells, followed by incubation in the presence of a subject substance, for a given period of time, after which cells are extracted or the supernatant is recovered, and the resulting product is quantified by a method appropriate thereto. When it is difficult to detect the production of the cell-stimulating activity index substance due to a degradation enzyme contained in the cells, an inhibitor against said degradation enzyme may be added before assay. The operation activity of the subject substance can be assessed by measuring an activity via an aberrant gene product (e.g., growth promotion, promotion or suppression of intracellular protein phosphorylation), or the change in the responding substance concentration in the intracellular signal transduction system (e.g. CAMP, inositol phosphate, calcium ion, or the like), when a subject substance is brought into contact with cells containing said product, or by measuring an activity (e.g., growth promotion, promotion or suppression of intracellular protein phosphorylation) via an aberrant gene product, or the change in responding substance concentration in the intracellular signal transduction system (e.g. cAMP, inositol phosphate, calcium ion, or the like), when a subject substance is brought into contact with said aberrant receptor expressed on the cell membrane of a transformant containing DNA encoding the aberrant receptor by culturing said transformant.

Also, the present invention provides a method of creating a drug for treatment of a disease caused by an aberrant gene product, wherein the method comprises bringing the aberrant gene product (e.g., aberrant receptor) is into contact with a subject substance, assessing the operation activity of said substance on said product, and preparing a drug judged to substantially operate the aberrant gene product. The present invention also provides a method of preparing a drug for treatment of a disease caused by an aberrant gene product, wherein the method comprises bringing an aberrant receptor into contact with a subject substance assessing the operation activity of said substance on said aberrant receptor, and preparing a drug judged to substantially operate the signal transduction system of a cell containing the aberrant receptor. Thus, a new method of pharmaceutical development or preparation is established based on information obtained from analysis of structural genes associated with human diseases, which serves to elucidate the causal relationship between a gene mutation and a disease.

The drug thus created can, for example, be used orally in the form of tablets, capsules, elixirs, microcapsules etc., all of which may be sugar coated as necessary, or non-orally in the form of injectable preparations such as aseptic solutions and suspensions in water or other pharmaceutically acceptable liquids. Also, the desired prophylactic/therapeutic agent can be produced by mixing the drug thus obtained with physiologically acceptable carriers, flavoring agents, excipients, vehicles, antiseptics, stabilizers, binders etc. in unit dosage forms for generally accepted manners of pharmaceutical making.

Additives which can be mixed in tablets, capsules etc. include, for example, binders such as gelatin, corn starch, tragacanth and gum arabic, excipients such as crystalline cellulose, swelling agents such as corn starch, gelatin and alginic acid, lubricants such as magnesium stearate, sweetening agents such as sucrose, lactose and saccharin, and flavoring agents such as peppermint, and cherry. When the unit dosage form is the capsule, the above-mentioned materials may further incorporate liquid carriers such as oils and fats. Sterile compositions for injection can be formulated by ordinary methods of pharmaceutical making such as by dissolving or suspending active ingredients, naturally occurring vegetable oils such as sesame oil and coconut oil, etc. in vehicles such as water for injection. Aqueous liquids for injection include physiological saline and isotonic solutions containing glucose and other auxiliary agents (e.g., D-sorbitol, D-mannitol, sodium chloride), and may be used in combination with appropriate dissolution aids such as alcohols (e.g., ethanol), polyalcohols (e.g., propylene glycol, polyethylene glycol), nonionic surfactants (e.g., polysorbate 80 (trade name), HCO-50) etc. Oily liquids include sesame oil and soybean oil, and may be used in combination with dissolution aids such as benzyl benzoate and benzyl alcohol.

The aqueous liquid may also be formulated with buffers (e.g., phosphate buffer, sodium acetate buffer), soothing agents (e.g., benzalkonium chloride, procaine hydrochloride), stabilizers (e.g., human serum albumin, polyethylene glycol), preservatives (e.g., benzyl alcohol, phenol), antioxidants etc. The thus-prepared injectable liquid is normally filled in an appropriate ampule. Because the thus-obtained preparation is effective even at low doses, and therefore safe and of low toxicity, it can be administered to warm-blooded mammals (e.g., rats, rabbits, sheep, pigs, bovines, cats, dogs, monkeys, humans), for instance. The dose is normally about 0.1 mg to 100 mg, preferably about 1.0 to 50 mg, and more preferably about 1.0 to 20 mg per day for an adult (weighing 60 kg) in oral administration as a therapeutic agent for hypertension, depending on target disease, symptoms etc. In non-oral administration, it is advantageous to administer the drug in the form of injectable preparation at a daily dose of about 0.01 to 30 mg, preferably about 0.1 to 20 mg, and more preferably about 0.1 to 10 mg per administration for an adult (weighing 60 kg), depending on subject of administration, target organ, symptoms, method of administration etc. For other animal species, corresponding doses as converted per 60 kg weight can be administered.

Furthermore, the present invention provides a use of a substance capable of operating an aberrant gene product for treatment of a disease caused by said aberrant gene product (e.g., aberrant receptor), a use of an aberrant gene product obtained by expressing in a cell the gene encoding the aberrant gene product (e.g., aberrant receptor) for screening for a substance for treatment of a disease caused by said product, a use of an aberrant gene product obtained by expressing in a cell the gene encoding the aberrant gene product (e.g., aberrant receptor) for screening for an operating substance for said product, etc.

### Best Mode for Carrying out the Invention

Abbreviations for bases, amino acids and others used in the present specification are based on abbreviations specified by the IUPAC-IUB Commission on Biochemical Nomenclature or abbreviations in common use in relevant fields. Some examples are given below. When an optical isomer may be present in amino acid, it is of the L-configuration, unless otherwise stated.
- DNA :: Deoxyribonucleic acid
- A :: Adenine
- T :: Thymine
- G :: Guanine
- C :: Cytosine
- SDS :: Sodium dodecyl sulfate
- Gly :: Glycine (G)
- Ala :: Alanine (A)
- Val :: Valine (V)
- Leu :: Leucine (L)
- Ile :: Isoleucine (I)
- Ser :: Serine (S)
- Thr :: Threonine (T)
- Cys :: Cysteine (C)
- 1/2 Cys:: Half-cystine
- Met :: Methionine (M)
- Glu :: Glutamic acid (E)
- Asp :: Aspartic acid (D)
- Lys :: Lysine (K)
- Arg :: Arginine (R)
- His :: Histidine (H)
- Phe :: Phenylalanine (F)
- Tyr :: Tyrosine (Y)
- Trp :: Tryptophan (W)
- Pro :: Proline (P)
- Asn :: Asparagine (N)
- Gln :: Glutamine (Q)
- Apr :: Ampicillin resistance gene
- Ter :: Tetracycline resistance gene

The present invention is hereinafter described in more detail by means of the following examples, which only serve for exemplification and are not to be construed as limitative to the present invention.

### Example 1

### Cloning of human β3 adrenergic receptor cDNA

To amplify human β3 adrenergic receptor cDNA by the PCR method, the following two primers (Nos. 1 and 2) were synthesized, with reference to a known nucleotide sequence of the human β3 adrenergic receptor [J.M. Lelias et al.,. FEBS Lett., Vol. 324, p. 127 (1993)].

Sense primer No. 1:
5'-ATTTGGAGACCCCCTCCTTCCTTCTTTCC-3'
(SEQ ID NO:1)

Antisense primer No. 2:
5'-ACAGAGTTGTTGCTTCTTGTCCTTCAGGCC-3'
(SEQ ID NO:2)

Using Takara Ex Taq (Takara Shuzo Co., Ltd.) and the included buffer, the following PCR reaction was carried out. Using 0.5 *µ*l of a human fat cell-derived cDNA library (CLONTECH Laboratories, Inc.) as the template, 5 *µ*l of the attached PCR reaction buffer (10 fold diluted), 4 *µ*l of a dNTP mixture, 0.5 *µ*l (2.5 U) of TaKaRa Ex Taq, and each of 2 kinds of primers (combinations of primer No. 1 above and the λgt11 forward primer (Takara Shuzo Co., Ltd.) and of primer No. 1 above and the λgt11 reverse primer (Takara Shuzo Co., Ltd.); each 100 pmol) were added to a tube and diluted to 50 *µ*l with sterile distilled water. A PCR reaction was carried out at 94°C for 2 minutes, 61°C for 1 minute and 72°C for 1 minute in 25 cycles; the 2 tubes were mixed; to 5 *µ*l of this reaction mixture, 2 primers (Nos. 1 and 2 above; each 100 pmol) were added, followed by a PCR reaction at 94°C for 2 minutes, 55°C for 1 minute and 72°C for 1 minute in 30 cycles. When the PCR product was separated by 1% agarose gel electrophoresis, an amplified DNA fragment was confirmed at a position corresponding to the size (1310 bp) expected from the nucleotide sequence of the human β3 adrenergic receptor. This DNA fragment was recovered from the agarose gel and inserted into the SmaI site of the plasmid vector pUC19 (Takara Shuzo Co., Ltd.) to yield p19-hβ3R. The nucleotide sequence of the cDNA was determined by the dideoxynucleotide synthetic chain termination method [T. Messing et al., Nucl. Acids Res., Vol. 9, p. 309 (1981)] and confirmed as identical to the published sequence.

### Example 2

### Preparation of human β3 adrenergic receptor mutant (Trp64Arg) DNA

A synthetic oligonucleotide for mutagenesis was synthesized, and a mutant was generated using the Mutan-Super Express Km (Takara Shuzo Co., Ltd.) as follows: Oligonucleotide for mutagenesis No. 3:

5'-TGGCCATCGCCCGGACTCCGAG-3'
(SEQ ID NO:3)

A DNA fragment obtained by digestion of the plasmid p19-hβ3R described in Example 1 with restriction enzymes EcoRI and XbaI was recovered from agarose gel and inserted between the EcoRI and XbaI sites of the plasmid vector pKF18k attached to the kit (Takara Shuzo Co., Ltd.) to yield p18k-hβ3R. Using 10 ng of this DNA as the template, in the presence of 5 pmol of the attached selection primer, 5 pmol of No. 5 primer above, 5 *µ*l of a reaction buffer (10 fold diluted), 4 *µ*l of a dNTP mixture, and 0.5 *µ*l (2.5 U) of TaKaRa LA Taq, diluted to 50 *µ*l with sterile distilled water, a PCR reaction was carried out at 94°C for 1 minute, 55°C for 1 minute and 72°C for 3 minutes in 25 cycles, followed by DNA recovery by ethanol precipitation. This DNA was distilled in 5 *µ*l of sterile distilled water; using 2 *µ*l of this solution, the DNA was introduced into the attached *Escherichia coli* MV1184 strain to yield transformants capable of growing on kanamycin-containing (50 *µ*g/ml) LB plates. Of these transformants, several clones were subjected to nucleotide sequencing at the mutation site; a clone confirmed as having the mutation introduced was designated p18k-hβ3(W64R)R.

### Example 3

### Preparation of recombinant DNA for expression of the human β3 adrenergic receptor gene in animal cells

After the plasmid p19-hβ3R described in Example 1 was digested with EcoRI and Xbal, a human β3 adrenergic receptor cDNA fragment was recovered from agarose gel. Next, the above-described cDNA fragment was inserted between the EcoRI and XbaI sites of pME18S [R. Sasada et al., Biochem. Biophys. Res. Commun., Vol. 190, p. 1,173 (1993)], a vector for transient expression in animal cells, to yield the expression plasmid phβ3R201. Next, to select a cell line showing stable expression, the neomycin resistance gene (neo), a drug resistance marker, was inserted, as described below. First, a fragment consisting of the SV40 early promoter, the neo gene and the SV40 polyadenylation signal was inserted between the KpnI and SspI sites of the plasmid phβ3R201 to yield the plasmid phβ3R203.

After the plasmid p18k-hβ3(W64R)R described in Example 2 was digested with EcoRI and Xbal, a human β3 adrenaline receptor variant cDNA fragment was recovered from agarose gel. Following the same procedures as those shown above, the plasmids phβ3(W64R)R201 and phβ3(W64R)R203 were prepared.

### Example 4

### Expression of the human β3 adrenergic receptor gene in animal cells

The plasmids described in Example 3 (phβ3R203 and phβ3(W64R)R203) were each introduced into CHO cells (Chinese hamster ovary cells) using the Mammalian Transfection Kit (STRATAGENE, CA, USA) as follows:

To an 80 cm² flask, 15 ml of a complete medium [Ham F-12 medium containing 10% (v/v) FCS (fetal calf serum) (LIFE TECHNOLOGIES, INC.)] was added, and 5 × 10⁵ CHO cells were seeded. After overnight cultivation in the presence of 5% carbon dioxide at 37°C, this culture medium was replaced with 10 ml of the above medium. After sterile water was added to 30 *µ*g of the plasmid described in Example 1 (phβ3R203) to make 450 ml, 50 *µ*l of solution #1, then 500 *µ*l of solution #2, both included in the kit, were mixed in the 450 ml. After this mixture was kept standing at room temperature for 15 minutes, 1 ml aliquot was added drop by drop onto, and mixed with, the cells, followed by overnight cultivation in the presence of 3% carbon dioxide at 37°C. On the following day, the medium was removed; after the cell surface was washed with F-12 medium (serum-free), 15 ml of a complete medium (10% FCS/F-12) was added, followed by overnight cultivation in the presence of 5% carbon dioxide at 37°C. On the following day, the above cells were seeded at about 200 cells per well to a 12-well plate supplemented with a D-MEM-F-12 medium (Nikken Seibutsu Igaku Kenkyujo) containing 10% FCS, followed by overnight cultivation, after which the cells were further cultured in a medium containing 400 *µ*g/ml G418 (LIFE TECHNOLOGIES, INC.) (10% FCS/D-MEM/F-12) until colony formation was noted, with medium replacement every 3 to 4 days. After a large number of colonies were found to form, 1 to several colonies per well were seeded to, and cultured on, a 24-well plate supplemented with a 10% FCS/D-MEM/F-12 medium containing 400 *µ*g/ml G418 (2 ml/well), and G418-resistant cells were harvested (primary cloning). Next, for 2 to 3 wells of the cells harvested in the primary cloning, 10 colonies per well were sown to, and cultured on, a 24-well plate in the presence of G418 at an increased concentration of 800 *µ*g/ml, and G418-resistant cells were harvested. Out of these cells, a line showing high expression of the human β3 adrenergic receptor was selected by the method described in Example 5 below (secondary cloning).

### Example 5

### Determination of cAMP activity in cells showing expression of a human β3 adrenergic receptor

cAMP production activity was determined using the CAMP ELA SYSTEM (Amersham, UK) as follows:

Each of the culture media of cells showing expression of a human β3 adrenergic receptor obtained on a 24-well plate in Example 4 (CHO/phβ3R203 and CHO/phβ3(W64R)R203) was replaced with a G418-free medium (10% FCS/D-MEM/F-12); 0.5 mM IBMX (3-isobutyl-1-methylxanthine, Wako Pure Chemical Industries) was added. After cultivation in the presence of 5% carbon dioxide at 37°C for 30 minutes, 10-5 isoproterenol [(±)-isoproterenol, Funakoshi] was added, followed by further cultivation for 30 minutes. Next, the cell surface was three times washed with 4°C PBS (phosphate-buffered saline); 300 *µ*l of 0.1 N hydrochloric acid was added, followed by boiling at 95°C for 10 minutes. A 25 *µ*l aliquot was collected from each well and dissolved in 75 *µ*l of the assay buffer attached to the kit. After a 50 *µ*l aliquot of the solution, together with 100 *µ*l of an anti-cAMP rabbit antibody, was added to each well of the kit component 96-well microtiter plate with an anti-rabbit IgG donkey antibody immobilized thereon, the plate was kept standing at 4°C for 1 hour. Next, 100 *µ*l of HRP-labeled cAMP was added; after the plate was kept standing at 4°C for 1 hour, each well was washed 4 times, followed by the addition of 150 *µ*l of TMB (3,3,5,5-tetramethylbenzidine); after the plate was kept standing at room temperature for 60 minutes, 100 *µ*l of 1.0 N sulfuric acid was added to each well, followed by absorptiometry at 450 nm wavelength. A cell line showing increased cAMP production activity in the presence of isoproterenol, a β adrenergic receptor agonist, was selected.

### Example 6

### Determination of ligand binding activity of a mutant human β3 adrenergic receptor

A line of Chinese hamster ovary (CHO) cells having the Trp64Arg variant human β3 adrenergic receptor expressed therein were prepared for use in the search for compounds that specifically bind to the Trp64Arg variant human β3 adrenaline receptor. After being three times washed with an ice-cooled phosphate buffer, the CHO cells having the Trp64Arg variant human β3 adrenergic receptor expressed therein were immersed in an ice-cooled hypotonic solution (1 mM Tris-HCl buffer, pH 7.2) for 10 minutes; these after the cells were detached, followed by centrifugation at 4°C and 18,000 rpm for 15 minutes and recovery of the cell membrane fraction, which contained said receptor. After TME buffer (75 mM Tris-HCl buffer, pH 7.4, 12.5 mM magnesium chloride, 1.5 mM EDTA, 4 *µ*M desipramine, 5 *µ*g/ml. leupeptine, 1 *µ*g/ml benzamidine, 5 *µ*g/ml trypsin inhibitor and 40 *µ*g/ml basitlacine) was added, the cell membrane fraction obtained was uniformly suspended using a 25 gauge injection needle to yield a standard receptor preparation. The standard receptor preparation prepared (containing 10 to 30 *µ*g, based on protein), [¹²⁵I]-iodocyanopindrole (240 pM, DuPont-NEN, USA) and the subject compound were mixed; after adjustment to a final volume of 250 *µ*l with TME buffer, the mixture was kept standing at room temperature for 90 minutes. The mixture was aspirated and filtered using a glass fiber filter (GF/B, Packard Instrument Co., Inc., USA) and a cell harvester (Filter Mate Cell Harvester, Packard Instrument Co., Inc., USA); the radioactivity in the filter was determined using a scintillation counter (TopCount Microplate Scintillation Counter, Packard Instrument Co., Inc., USA). Non-specific binding was quantified in the presence of (S)-(-)-propranolol (Sigma, USA) added to a final concentration of 100 *µ*M.

### Example 7

### Determination of cAMP-increasing activity in CHO cells expressing a mutant human β3 adrenergic receptor

A line of Chinese hamster ovary (CHO) cells having the Trp64Arg variant human β3 adrenergic receptor expressed therein were seeded to a 96-well microtiter plate (1 × 10⁴ cells/well); after 72 hours of cultivation following confluence, the subject compound was added; the plate was kept standing at 37°C for 40 minutes (100 *µ*l/well). After the cells were thrice washed with 4°C phosphate buffer, 0.1 N hydrochloric acid was added, followed by boiling at 95°C for 10 minutes. A 25 *µ*l aliquot was collected from each well and dissolved in 75 *µ*l of the assay buffer attached to the cAMP EIA SYSTEM (Amersham, UK); a 50 *µ*l sample was taken from the solution and quantified using the above CAMP EIA SYSTEM, as described below. After the above sample (50 *µ*l) and 100 *µ*l of an anti-cAMP rabbit antibody were added to a 96-well microtiter plate with an anti-rabbit IgG donkey antibody immobilized thereon, the plate was kept standing at 4°C for 2 hours; after 100 *µ*l of horse radish peroxidase (HRP)-labeled CAMP was added, the plate was kept standing at 4°C for 1 hour. After each well was aspirated, the plate was washed 4 times with a washing solution (400 *µ*l/well). Next, 150 *µ*l of tetramethylbenzidine, an HRP substrate, was added to each well, followed by 60 minutes of incubation during shaking at room temperature. Finally, 100 *µ*l of 1.0 N sulfuric acid was added to stop the reaction, after which cAMP was quantified by absorptiometry at 450 nm wavelength. The results obtained demonstrated that the cAMP concentration in CHO cells increased depending on the concentration of compound added, and that the increase was significant in comparison within the absence of the compound.

### Example 8

### Compound screening

According to compound screening in accordance with the method of Example 6, a compound showing high affinity for the Trp64Arg variant human β3 adrenergic receptor can be obtained. Testing a compound in accordance with the method of Example 7 reveals that the cAMP concentration in CHO cells showing expression of the Trp64Arg variant human β3. adrenergic receptor increase with its concentration.

All references mentioned herein are incorporated by reference. The invention has been described in detail with particular reference to preferred embodiments thereof. However, it will be appreciated that modifications and improvements with the spirit and teachings of the inventions may be made by those in the art upon considering the present disclosure.

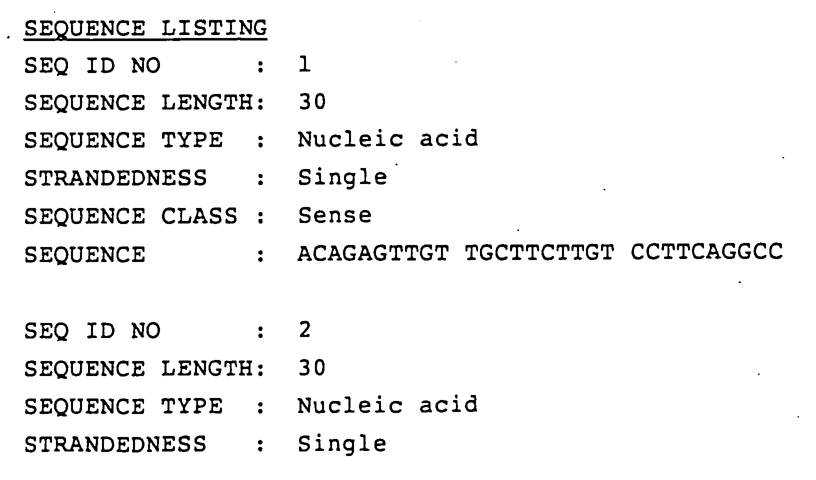

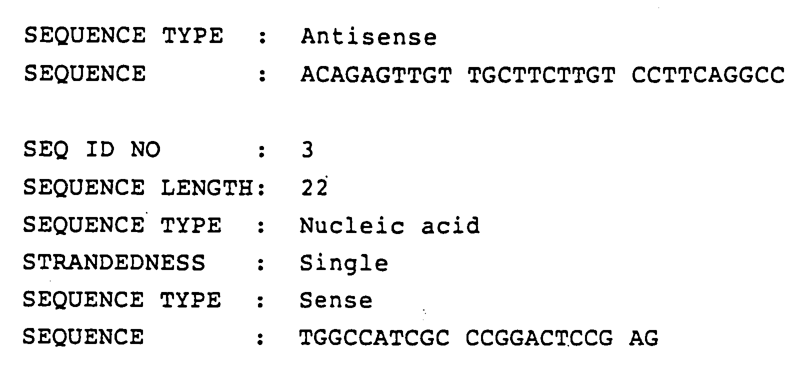

A pharmaceutical composition comprising a substance capable of causing an aberrant gene product to function in a manner similar to the wild-type gene product and a pharmaceutically acceptable carrier.

The pharmaceutical composition as defined above, wherein the substance is an agonist or an antagonist of the aberrant gene product.

The pharmaceutical composition as defined above, wherein the aberrant gene product is an aberrant receptor, an aberrant channel, an aberrant signal or an aberrant enzyme.

The pharmaceutical composition as defined above, wherein the aberrant gene product is an aberrant receptor and wherein the composition is used for prophylaxis and/or treatment of a disease caused by an aberrant receptor.

The pharmaceutical composition as defined above, wherein the disease caused by an aberrant receptor comprises a disease caused by substantial reduction in the activity of the signal transduction system of the cell having the aberrant receptor.

The pharmaceutical composition as defined above, wherein the disease caused by an aberrant receptor comprises a disease caused by the substantial absence of the action of a natural ligand on the signal transduction system of the cell having the aberrant receptor.

The pharmaceutical composition as defined above, wherein the disease caused by an aberrant receptor comprises a disease caused by substantial reduction in the affinity of a natural ligand for the aberrant receptor.

The pharmaceutical composition as defined above, wherein the signal transduction system comprises a signal transduction system based on the change in intracellular concentration of a responding substance resulting from the binding of a natural ligand and a receptor.

The pharmaceutical composition as defined above, wherein the signal transduction system further comprises the responding substance selected from the group consisting of cAMP, inositol phosphate(s) or calcium ion.

The pharmaceutical composition according to claim 4, wherein the substance capable of operating an aberrant receptor is a substance that operates the normal receptor. 11. The pharmaceutical composition as defined above, wherein the substance capable of operating an aberrant receptor is a substance that does not operate the normal receptor.

A method of treating a disease caused by an aberrant gene product in an animal, comprising providing to the animal an effective amount of a substance capable of operating the aberrant gene product.

The use as defined above, wherein the aberrant gene product is an aberrant receptor. 14. A method of screening for a substance capable of operating an aberrant gene product comprising bringing an aberrant gene product into contact with a subject substance and assaying the operation activity of said substance on said product.

The screening method as defined above, wherein the aberrant gene product is an aberrant receptor.

The screening method for a substance for treatment of a disease caused by an aberrant gene product comprising bringing an aberrant gene product into contact and a subject substance and assaying the operation activity of said substance on said product.

A method of screening for a drug for restoring normal function to a signal transduction system of a cell having an aberrant receptor of a mammal suffering from a disease caused by the aberrant receptor, which comprises bringing the aberrant receptor into contact with a subject substance and assaying the activity of said substance on said receptor and wherein the activity is an activity that restores the normal function of the cell.

The screening method as defined above, wherein the aberrant receptor is an aberrant receptor prepared by expressing in a cell the gene encoding the aberrant receptor.

The screening method as defined above, wherein the gene encoding the aberrant receptor is an aberrant receptor-encoding gene specified by comparative analysis of a gene prepared from a cell of a mammal suffering from a disease caused by the aberrant receptor, and a gene prepared from a cell of a mammal of the same species that does not carry the aberrant receptor.

A method of preparing a drug for treatment of a disease caused by an aberrant gene product, which comprises bringing the aberrant gene product into contact with a subject substance, assaying the activity of said substance on said product and preparing a substance judged to substantially operate the signal transduction system of a cell having the aberrant gene product wherein said activity is activity that restores wide-type activity of the gene product.

A method of preparing a substance for treatment of a disease caused by an aberrant receptor, which comprises bringing the aberrant receptor into contact with a subject substance, assaying the activity of said substance on the aberrant receptor and preparing a substance judged to substantially operate the signal transduction system of a cell having the aberrant receptor, wherein said activity is activity that restores wild-type activity to the receptor.

The method as defined above, wherein the aberrant receptor is an aberrant receptor prepared by expressing in a cell the gene encoding the aberrant receptor.

The method as defined above, wherein the gene encoding the aberrant receptor is an aberrant receptor-encoding gene specified by comparative analysis of a gene prepared from a cell of a mammal suffering from a disease caused by the aberrant receptor, and a gene prepared from a cell of a mammal of the same species that does not carry the aberrant receptor.

A method of screening for a substance capable of operating an aberrant gene product comprising expressing in a cell the gene encoding the aberrant gene product, separating the aberrant gene product, providing a substance as the aberrant gene product and determining operation activity of said substance as said gene product.

The method as defined above, wherein the aberrant gene product is an aberrant receptor.

The method as defined above, wherein the substance is a substance that normally operates said product.

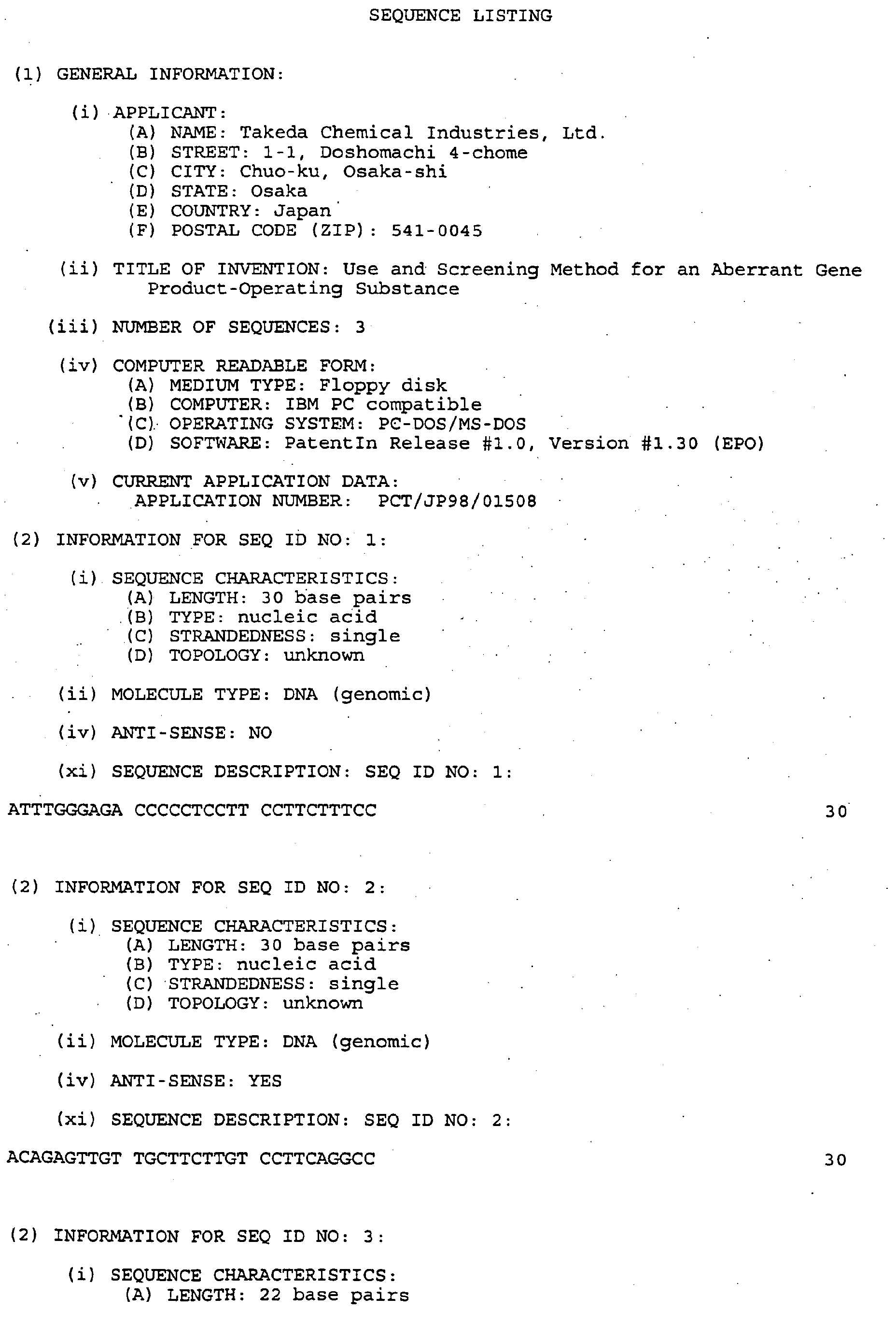

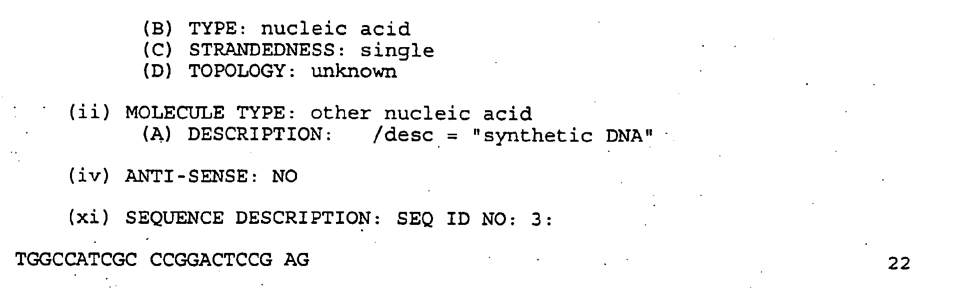

## Claims

1. A method of screening for a substance being capable of operating an aberrant gene product, comprising bringing the aberrant gene product into contact with a subject substance and assaying the operation activity of said substance on said product. [original claim 14]

2. The method according to claim 1, wherein the aberrant gene product is an aberrant receptor, an aberrant channel, an aberrant signal or an aberrant enzyme.

3. The method according to claim 1, wherein the aberrant gene product is an aberrant enzyme.

4. The method according to claim 1, wherein the aberrant gene product is an aberrant gene product prepared by expressing in a cell the gene encoding the aberrant gene product.

5. A method of screening for a substance for the treatment of a disease caused by an aberrant gene product, comprising bringing the aberrant gene product into contact with a subject substance and assaying the operation activity of said substance on said product.

6. The method according to claim 5, wherein the gene encoding the aberrant gene product is an aberrant gene product-encoding gene specified by comparative analysis of a gene prepared from a cell of a mammal suffering from a disease caused by the aberrant gene product, and a gene prepared from a cell of a mammal of the same species that does not carry the aberrant gene product.

7. A method of screening for a substance being capable of operating an aberrant gene product, comprising expressing in a cell the gene encoding the aberrant gene product, bringing the aberrant gene product into contact with a subject substance and assaying the operation activity of said substance on said product.

8. The method according to claim 7, wherein the aberrant gene product is an aberrant receptor, an aberrant channel, an aberrant signal or an aberrant enzyme.

9. The method according to claim 7, wherein the aberrant gene product is an aberrant enzyme.

10. The method according to claim 7, wherein the substance is a substance that normally operates said product.

11. A method of screening for a substance being capable of operating an aberrant enzyme being an enzyme with a mutation in the structural gene resulting in substantially changed affinity for an activator or inhibitor that operates a normal enzyme, comprising expressing in a cell the gene encoding the aberrant enzyme, bringing the aberrant enzyme into contact with a subject substance and assaying the operation activity of said substance on said enzyme.

12. The method according to claim 11, wherein the aberrant enzyme causes a disease due to the substantial change in the affinity of said activator or inhibitor, which further comprises selecting an activator or inhibitor to the aberrant enzyme.

13. The method according to claim 11, which is suitable for a drug for restoring normal response to a substrate protein of the aberrant enzyme and which comprises assaying the activity of said substance on said enzyme and wherein the activity is an activity that restores the normal response to the substrate protein of the aberrant enzyme.

14. The method according to claim 11, which is suitable for identifying a drug for the treatment of a disease caused by an aberrant enzyme, which comprises assaying the activity of said substance on said enzyme and preparing a substance judged to substantially operate response to a substrate protein of the aberrant enzyme wherein said activity is an activity that restores wild-type activity of the enzyme.

15. The method according to claim 13, wherein the aberrant enzyme is an aberrant enzyme prepared by expressing in a cell the gene encoding the aberrant enzyme.

16. The method according to claim 15, wherein the gene encoding the aberrant enzyme is an aberrant enzyme-encoding gene specified by comparative analysis of a gene prepared from a cell of a mammal suffering from a disease caused by the aberrant enzyme, and a gene prepared from a cell of a mammal of the same species that does not carry the aberrant enzyme.

17. The method according to claim 11, which is suitable for screening an activator or inhibitor of the aberrant enzyme and which comprises expressing in a cell the gene encoding the aberrant enzyme, separating aberrant enzyme, providing a substance with the aberrant enzyme and determining operation activity of said substance as said enzyme.

18. The method according to claim 17, wherein the substance is a substance that normally operates said enzyme.
